Europäisches Patentamt

European Patent Office

Office européen des brevets

(19)

(11) Numéro de publication : **0 010 013**
**B1**

(12) ## FASCICULE DE BREVET EUROPÉEN

(45) Date de publication du fascicule du brevet :
21.10.81

(51) Int. Cl.³ : **C 07 C 31/36, C 07 C 29/64**

(21) Numéro de dépôt : 79400606.4

(22) Date de dépôt : 04.09.79

(54) **Procédé de préparation de la chlorhydrine éthylénique.**

(30) Priorité : 29.09.78 FR 7827892

(43) Date de publication de la demande :
16.04.80 (Bulletin 80/08)

(45) Mention de la délivrance du brevet :
21.10.81 Bulletin 81/42

(84) Etats contractants désignés :
BE DE FR GB IT NL

(56) Documents cités :

CHEMICAL ABSTRACTS, vol. 54, 25 novembre 1960, n° 22, réf. 24376b, Columbus, Ohio, US.
M. REPAS et al. : «Preparation of anhydrous ethylene chlorohydrin».

CHEMICAL ABSTRACTS, vol. 74, 1971, 10 mai 1971, n° 19, page 456, ref. 99387u, Columbus, Ohio, US
PARAUSANU et al. : Synthesis of ethylene chlorohydrin by the hydrochlorination of ethylene oxide in the liquid phase»

THEORETICA CHIMICA ACTA, vol. 51, n° 1, 1979, Berlin (SPRINGER INTERNATIONAL)
«A theoretical ab initio study of the formation of 2 fluoro-ethanol from oxirane and HF», pages 11-35, éditeurs G. ALAGONA et al.

(73) Titulaire : **P C U K PRODUITS CHIMIQUES UGINE KUHLMANN**
**Service Propriété Industrielle Tour Manhattan**
**F-92087 PARIS LA DEFENSE 2 Cédex 21 (FR)**

(72) Inventeur : **Jequier, William**
**35bis, Boulevard Saint-André**
**F-60000 Beauvais (FR)**
Inventeur : **Ghenassia, Elie**
**100, rue Benoîte Vincent**
**F-62400 Bethune (FR)**
Inventeur : **Fine, François**
**Place de l'Eglise**
**F-69720 Saint Laurent de Mure (FR)**
Inventeur : **Krempf, Gérard, René**
**2, rue Nicolas Berthet**
**F-69110 Sainte Foy Les Lyon (FR)**

(74) Mandataire : **Houssin, Jean et al**
**PRODUITS CHIMIQUES UGINE KUHLMANN Service Propriété Industrielle Tour Manhattan Cedex 21**
**F-92087 Paris La Defense 2 (FR)**

Imprimerie Jouve, 17, rue du Louvre, 75001 Paris, France

Procédé de préparation de la chlorhydrine éthylénique

La présente invention concerne un procédé de préparation de la chlorhydrine éthylénique, ou chloro 2 éthanol, de formule $CH_2OH-CH_2Cl$, à partir d'oxyde d'éthylène et d'acide chlorhydrique dans des conditions telles que l'acide chlorhydrique excédentaire et la chlorhydrine éthylénique formée restent gazeux dans le réacteur pendant la réaction.

La chlorhydrine éthylénique, produit intermédiaire dans la fabrication des élastomères polysulfurés tels que les thiocols, est utilisée également dans la synthèse de certains pesticides, de peintures et vernis, ou encore comme solvant dans la préparation de l'acétyl-cellulose.

Dans le brevet français 1.511.682 est décrit un procédé de préparation de la chlorhydrine éthylénique à partir de l'éthylène et du chlore en milieu aqueux. Ce procédé présente l'inconvénient de donner une solution diluée dont il est difficile et onéreux d'extraire la chlorhydrine éthylénique sous forme anhydre du fait de la formation d'un azéotrope à 58 % d'eau — 42 % de chlorhydrine éthylénique et de point d'ébullition de 97,8 °C.

Une autre technique de préparation de la chlorhydrine éthylénique, décrite dans le brevet allemand 968.902, consiste à faire passer un gaz contenant de l'oxyde d'éthylène dans de la chlorhydrine éthylénique saturée d'acide chlorhydrique. Cette façon d'opérer entraîne une formation importante de produits supérieurs : chlorhydrine du diéthylène glycol (CHDEG) $CH_2OH-CH_2-O-CH_2-CH_2Cl$ et chlorhydrine de triéthylène glycol (CHTEG) $CH_2OH-CH_2-O-CH_2-CH_2-O-CH_2-CH_2Cl$ ; aussi le rendement en chlorhydrine éthylénique (CHEG) ne dépasse-t-il pas 80-90 %.

Le certificat d'auteur russe 422.241 cite avec ses inconvénients un procédé de préparation de chlorhydrine éthylénique par réaction en phase liquide de l'oxyde d'éthylène avec l'acide chlorhydrique préalablement purifié. Selon la technique décrite dans ce certificat d'auteur russe 422.241, on remédie aux inconvénients de ce procédé en faisant réagir l'oxyde d'éthylène et l'acide chlorhydrique humide en présence d'organosilane. Cette technique ne permet cependant pas de dépasser des rendements en chlorhydrine éthylénique de 88-92 %.

Dans l'article extrait de CHEMICKY PRÛMYSL 10/35 (1960) pages 238-240, M. REPAS et J. PSCHERA décrivent un procédé de fabrication de chlorhydrine éthylénique à partir d'oxyde d'éthylène gazeux et d'acide chlorhydrique gazeux. Toutefois, la réaction s'effectuant à 30 °C en phase liquide ne permet pas d'obtenir des rendements supérieurs à 93 %.

Le procédé selon l'invention permet non seulement de limiter considérablement les inconvénients rencontrés dans les techniques de l'art antérieur, mais encore d'obtenir un rendement molaire minimum de 95 % en chlorhydrine éthylénique pure par rapport à l'oxyde d'éthylène et un rendement pondéral minimum de 97 %, le taux de conversion de l'oxyde d'éthylène atteignant 100 %. Ce procédé de préparation de la chlorhydrine éthylénique à partir de l'oxyde d'éthylène et de l'acide chlorhydrique se caractérise en ce que l'on fait réagir dans un réacteur l'oxyde d'éthylène à l'état gazeux sur l'acide chlorhydrique sec gazeux, le milieu réactionnel étant également maintenu à l'état gazeux pendant la réaction.

Selon un mode pratique de réalisation du procédé, l'oxyde d'éthylène utilisé est vaporisé à l'entrée du réacteur par passage dans un vaporisateur ou dans une zone de vaporisation avant d'entrer en contact avec l'acide chlorhydrique. La température de l'oxyde d'éthylène se trouve dans ces conditions à une température comprise entre 50 et 180 °C et de préférence comprise entre 50 et 120 °C. De même l'acide chlorhydrique pur et sec est introduit dans le réacteur à une température comprise entre 50 et 180 °C et de préférence comprise entre 50 et 120 °C. L'acide chlorhydrique peut être mis en réaction sous forme concentrée ou diluée dans un gaz inerte tel que par exemple l'azote, le gaz carbonique, l'hélium. La dilution de l'acide chlorhydrique dans un gaz inerte n'influence en rien la bonne mise en œuvre du procédé, elle permet cependant d'utiliser tel quel le mélange acide chlorhydrique — gaz inerte provenant par exemple de la synthèse du chlorure d'hydrogène à partir des brûleurs à chlore.

La réaction de l'oxyde d'éthylène sur l'acide chlorhydrique s'effectue généralement à une température dans le réacteur comprise entre 130 et 300 °C, de préférence comprise entre 130 et 250 °C et à une pression telle que la chlorhydrine éthylénique formée reste sous forme gazeuse. Cette pression d'utilisation est fonction des domaines de températures et de rapports molaires envisagés et déterminée par le point de rosée des mélanges des gaz en sortie du réacteur, c'est-à-dire pour un domaine de pression inférieure à 20 bars.

Le rapport molaire acide chlorhydrique sur oxyde d'éthylène est choisi en fonction de la productivité désirée. Il peut varier dans une large gamme et n'influe ni sur la sélectivité, ni sur le rendement de la réaction calculée à partir de l'oxyde d'éthylène. Il est cependant intéressant de se placer dans un rapport molaire tel que l'excès d'acide chlorhydrique, par simple échauffement de la masse, joue le rôle de régulateur de la réaction en éliminant les calories fournies par ladite réaction, de façon à obtenir la température convenable pour que le réacteur puisse fonctionner en adiabatique. Pratiquement le rapport molaire acide chlorhydrique sur oxyde d'éthylène peut varier de la stoechiométrie à 40 mais il est conseillé d'opérer entre 2 et 20 et mieux entre 2 et 10.

Le procédé peut également fonctionner en semi-isotherme dans un réacteur équipé d'un dispositif d'évacuation des calories par évaporation d'eau suivie de récupération de la chaleur produite.

Selon un des modes préférés d'exécution du procédé objet de l'invention, la chlorhydrine éthylénique peut être obtenue selon le cycle réactionnel schématisé en annexe. L'acide chlorhydrique gazeux et l'oxyde d'éthylène préchauffés sont introduits respectivement par les tubulures 1 et 2 à co-courant dans un réacteur 3 de type connu à la température et à la pression de réaction choisies. La chlorhydrine éthylénique à l'état gazeux et l'acide chlorhydrique, plus ou moins en excès, sortant du réacteur, sont refroidis par passage sur un échangeur 4, puis recueillis dans une colonne séparatrice ou un condenseur 5 duquel la fraction d'acide chlorhydrique non soluble dans la chlorhydrine éthylénique est éliminée par la tubulure 6. Le mélange chlorure d'hydrogène dissous et chlorhydrine éthylénique passe ensuite dans une colonne de distillation 7 d'où l'on extrait la chlorhydrine éthylénique pure, après en avoir chassé l'acide chlorhydrique, les produits lourds étant récupérés en fond de colonne. L'acide chlorhydrique récupéré dans les étapes de purification de la chlorhydrine éthylénique peut être recyclé au réacteur en 1.

Les exemples suivants illustrent le procédé précédemment décrit.

Exemple 1.

Dans un tube en verre à double enveloppe de diamètre intérieur 45 mm et de longueur 590 mm rempli d'anneaux Raschig en grès 6 × 6, le volume utile étant de 345 cm³, on fait arriver par le haut du réacteur à co-courant 93 g/h d'oxyde d'éthylène gazeux passé préalablement dans un vaporisateur à 50 °C et 274 g/h d'acide chlorhydrique gazeux préchauffé à 50 °C. La température est maintenue dans le réacteur à 155-160 °C à l'aide d'une circulation de fluide caloporteur permettant d'éliminer les calories de la réaction. On opère à pression atmosphérique, le temps de contact des réactifs étant de 3,6 secondes.

On recueille après passage dans un condenseur maintenu à 17 °C un mélange contenant 166,8 g de chlorhydrine brute et 26,0 g d'acide chlorhydrique dissous, 174 g d'HCl sont éliminés en tête du condenseur.

L'analyse chromatographique de la chlorhydrine brute après stripping de HCl dissous à 100-110 °C donne la répartition pondérale : 161,6 g de $CH_2OH-CH_2Cl$, 3,5 g $CH_2Cl-CH_2-O-CH_2-CH_2OH$ et 1,7 g de $CH_2Cl-CH_2-O-CH_2-CH_2-O-CH_2-CH_2OH$. Le taux de transformation de l'oxyde d'éthylène en chlorhydrine éthylénique est de 96,5 % et de 3,5 % en produits supérieurs : CHDEG et CHTEG. La conversion de l'oxyde d'éthylène est totale.

Exemple 2.

On opère dans le même appareillage que dans l'exemple 1, à pression atmosphérique, le temps de séjour étant de 3,6 secondes et le rapport molaire HCl/oxyde d'éthylène de 9 contre 3,6 dans l'exemple 1.

Dans le réacteur, on fait arriver à co-courant 44,8 g/h d'oxyde d'éthylène gazeux préchauffé à 60 °C et 338 g/h d'acide chlorhydrique gazeux et sec à 60 °C. La température dans le réacteur est maintenue à 133-136 °C, en éliminant les calories produites, à l'aide d'un fluide caloporteur.

On recueille après passage dans un condenseur maintenu à 18 °C un mélange contenant 81,4 g de chlorhydrine brute et 12,7 g d'HCl dissous. L'analyse chromatographique après élimination par chauffage de HCl dissous donne 72,8 g de chlorhydrine éthylénique, 1,86 g de CHDEG et 0,73 g de CHTEG. Le taux de transformation de l'oxyde d'éthylène en chlorhydrine éthylénique est de 95,8 %.

Exemple 3.

Dans un réacteur calorifugé, constitué par un tube vide de longueur 250 mm, de diamètre 22 mm et de volume 94 cm³, on envoie, par le haut du réacteur, à co-courant 52,9 g/h d'oxyde d'éthylène gazeux, 395 g/h d'acide chlorhydrique gazeux et sec et 42 g/h d'azote, les gaz étant tous les trois préchauffés à 60 °C. La température atteinte dans le réacteur sans l'aide d'aucun chauffage extérieur est de 162 °C. En opérant à pression atmosphérique la réaction est pratiquement instantanée. Le rapport molaire utilisé est de 9.

On recueille après passage dans un condenseur maintenu à 25 °C, 96,3 g de chlorhydrine brute et 15,1 g d'HCl dissous. Le mélange gazeux $N_2$ et HCl sortant en tête du condenseur, neutralisé par une solution alcaline, contient 336,5 g d'acide chlorhydrique. Le dosage de la chlorhydrine éthylénique brute, après avoir chassé HCl donne 94,4 g de CHEG, 1,7 g de CHDEG et 0,15 g de CHTEG. Le taux de transformation de l'oxyde d'éthylène en chlorhydrine éthylénique est de 97,6 % et celui en produits supérieurs de 2,4 %. La conversion de l'oxyde d'éthylène atteint 100 %.

Exemple 4.

Dans le même appareillage que celui utilisé dans l'exemple 2 on envoie à co-courant 69,4 g/h d'oxyde d'éthylène gazeux, 380 g/h d'acide chlorhydrique gazeux et sec et 47 g/h d'azote, les gaz étant préchauffés à 60 °C. La température atteinte dans le réacteur, sans l'aide d'aucun chauffage extérieur, est de 160 °C. Le rapport molaire HCl/oxyde d'éthylène est de 6,6 et le temps de séjour identique à celui de l'exemple 3, soit 0,7 seconde. On opère à pression atmosphérique.

On recueille après passage dans un condenseur maintenu à 25 °C, 147,3 g d'un mélange contenant 125,8 g de chlorhydrine éthylénique brute et 21,5 g d'HCl dissous. Le mélange gazeux $N_2$ + HCl sortant en tête du condenseur contient 302,3 g d'HCl. L'analyse chromatographique de la chlorhydrine brute donne 121,9 g de chlorhydrine éthylénique pure, 3,5 g de CHDEG et 0,4 g de CHTEG. Le taux de transformation en CHEG par rapport à l'oxyde d'éthylène est de 96 % et celui en termes supérieurs de 4 %. La conversion de l'oxyde d'éthylène est de 100 %.

Exemple 5.

Dans un réacteur pratiquement adiabatique constitué par un tube vide de diamètre 29 mm et de longueur 600 mm, on introduit à co-courant, par le haut du réacteur et à pression atmosphérique, 66,1 g/h d'oxyde d'éthylène gazeux, 378 g/h d'acide chlorhydrique gazeux recyclé, et 28 g/h d'azote, les réactifs étant préchauffés à 60 °C.

La température atteinte dans le réacteur est de 152 °C en dessous de la zone d'introduction des réactifs et 174-180 °C dans le reste de la zone réactionnelle. Le rapport molaire est de 6,8 et le temps de séjour de 3 secondes.

On recueille après passage dans un condenseur à 25 °C, 141 g d'un mélange contenant 120 g de chlorhydrine éthylénique brute et 21 g d'HCl dissous, le mélange $N_2-HCl$ sortant en tête du condenseur contient 299,1 g d'acide chlorhydrique. L'analyse de la chlorhydrine brute par chromatographie donne 47,2 g de chlorhydrine pure, 2,2 g de CHDEG et 0,6 g de CHTEG. Le taux de transformation en chlorhydrine éthylénique par rapport à l'oxyde d'éthylène est de 96,9 % et celui des termes supérieurs de 3,1 %.

Exemple 6.

Le réacteur utilisé dans l'exemple 5 est rempli d'anneaux Raschig 5 × 5 dans le but de parfaire le contact gaz-gaz. Le volume utile du réacteur devient alors 240 cm³.

On introduit à co-courant, par le haut du réacteur et à pression atmosphérique, 92 g/h d'oxyde d'éthylène gazeux, 572 g/h d'acide chlorhydrique gazeux recyclé et 63 g/h d'azote. Les réactifs sont préchauffés à 60 °C. La température atteinte dans le réacteur est de 138 °C en dessous de la zone d'introduction et 197-200 °C dans le reste de la zone réactionnelle. Le rapport molaire est de 7,4 et le temps de séjour de 1,2 seconde.

On recueille après passage dans un condenseur à 25 °C, 194,2 g d'un mélange contenant 167,8 g de chlorhydrine brute et 26,4 g d'acide chlorhydrique dissous. L'analyse de la chlorhydrine brute donne 163,9 g de chlorhydrine éthylénique pure, 3 g de chlorhydrine diéthylénique et 0,9 g de chlorhydrine triéthylénique. Le taux de transformation de l'oxyde d'éthylène en chlorhydrine éthylénique est de 97,4 %.

Exemple 7.

Dans le réacteur de l'exemple 6 en opérant à partir de 72,9 g/h d'oxyde d'éthylène gazeux et avec un rapport molaire HCl/oxyde d'éthylène de 8,85 à la température de 182-186 °C, le taux de transformation de l'oxyde d'éthylène en chlorhydrine éthylénique atteint 97,3 % pour une conversion totale de l'oxyde d'éthylène. Les réactifs sont introduits à co-courant par le haut du réacteur en opérant à pression atmosphérique, le temps de contact étant de 1,5 seconde.

Exemple 8.

On opère dans le même appareillage que dans l'exemple 2, la température de préchauffage étant portée à 120 °C. On introduit les mêmes quantités d'oxyde d'éthylène gazeux et d'acide chlorhydrique. La température est maintenue à 180 °C à l'aide d'une circulation de fluide caloporteur. Les rendements sont en tous points comparables à ceux de l'exemple 2.

Exemple 9.

Dans un tube en acier ordinaire de diamètre intérieur 21,2 mm et de longueur 300 mm, on introduit les mêmes quantités d'oxyde d'éthylène et d'acide chlorhydrique gazeux que dans l'exemple 2, mais sous une pression de 15 bars et préchauffées chacune à 120 °C. La température du réacteur est maintenue à 180 °C à l'aide d'une circulation de fluide caloporteur.

Les rendements sont en tous points comparables à ceux de l'exemple 2.

Exemple 10.

Dans un tube en acier ordinaire soigneusement calorifugé, constitué par un tube vide de longueur 250 mm et de diamètre 21,2 mm, on envoie à co-courant les mêmes quantités d'oxyde d'éthylène et d'acide chlorhydrique gazeux que pour l'exemple 3. Le préchauffage est également maintenu à 60 °C. On opère à une pression de 3 bars absolus. Les rendements sont en tous points comparables à ceux de l'exemple 3.

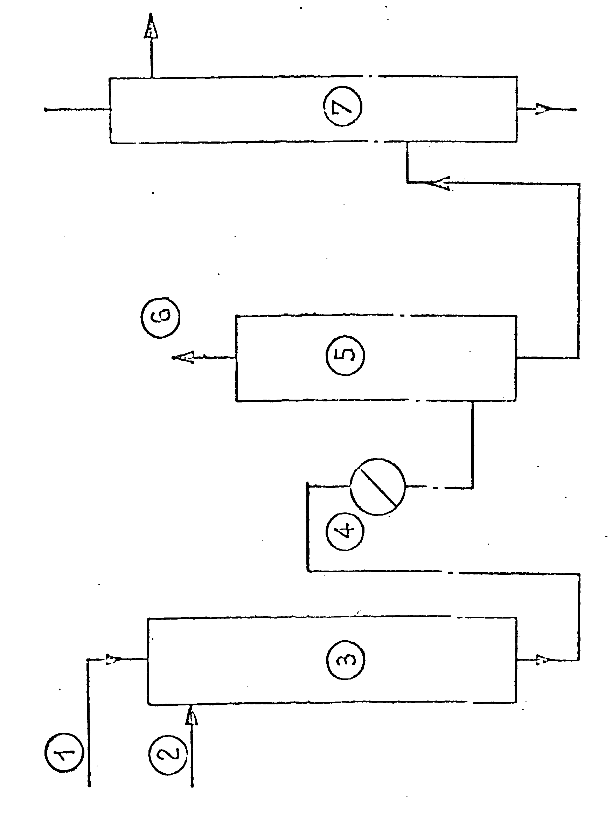

**Revendications**

1. Procédé de préparation de la chlorhydrine éthylénique par introduction dans un réacteur d'oxyde d'éthylène à l'état gazeux et d'acide chlorhydrique sec gazeux caractérisé en ce que, outre l'état gazeux de l'oxyde d'éthylène et de l'acide chlorhydrique pendant leur introduction, le milieu réactionnel est également maintenu à l'état gazeux pendant la réaction.

2. Procédé selon la revendication 1 caractérisé en ce que l'oxyde d'éthylène est introduit dans le milieu réactionnel à une température comprise entre 50 et 180 °C.

3. Procédé selon la revendication 1 caractérisé en ce que l'acide chlorhydrique est introduit dans le milieu réactionnel à une température comprise entre 50 et 180 °C.

4. Procédé selon l'une des revendications 1 à 3 caractérisé en ce que le rapport molaire acide chlorhydrique sur oxyde d'éthylène est compris entre la stœchiométrie et 40.

5. Procédé selon la revendication 4 caractérisé en ce que le rapport molaire acide chlorhydrique sur oxyde d'éthylène est compris entre 2 et 20.

6. Procédé selon l'une des revendications 1 à 5 caractérisé en ce que le milieu réactionnel est maintenu à une température comprise entre 130 et 300 °C.

7. Procédé selon la revendication 6 caractérisé en ce que le milieu réactionnel est maintenu à une température comprise entre 130 et 250 °C.

8. Procédé selon l'une des revendications 1 à 7 caractérisé en ce que le milieu réactionnel est maintenu sous une pression inférieure à 20 bars.

**Claims**

1. Process for the preparation of ethylene chlorohydrin by introducing, into a reactor, ethylene oxide in the gaseous state and dry gaseous hydrochloric acid, characterised in that, apart from the gaseous state of the ethylene oxide and of the hydrochloric acid during their introduction, the reaction medium is also kept in the gaseous state during the reaction.

2. Process according to Claim 1, characterised in that the ethylene oxide is introduced into the reaction medium at a temperature between 50 and 180 °C.

3. Process according to Claim 1, characterised in that the hydrochloric acid is introduced into the reaction medium at a temperature between 50 and 180 °C.

4. Process according to one of Claims 1 to 3, characterised in that the molar ratio of hydrochloric acid to ethylene oxide is between the stoechiometric ratio and 40.

5. Process according to Claim 4, characterised in that the molar ratio of hydrochloric acid to ethylene oxide is between 2 and 20.

6. Process according to one of Claims 1 to 5, characterised in that the reaction medium is kept at a temperature between 130 and 300 °C.

7. Process according to Claim 6, characterised in that the reaction medium is kept at a temperature between 130 and 250 °C.

8. Process according to one of Claims 1 to 7, characterised in that the reaction medium is kept under a pressure of less than 20 bars.

**Ansprüche**

1. Verfahren zur Herstellung von Äthylenchlorhydrin durch Einführen von gasförmigem Äthylenoxid und trockener, gasförmiger Chlorwasserstoffsäure in einem Reaktor, dadurch gekennzeichnet, daß man außer dem gasförmigen Zustand des Äthylenoxids und der Chlorwasserstoffsäure während ihrer Einführung auch das Reaktionsmedium während der Reaktion in gasförmigem Zustand hält.

2. Verfahren nach Anspruch 1, dadurch gekennzeichnet, daß das Äthylenoxid in das Reaktionsmedium bei einer Temperatur zwischen 50 und 180 °C eingeführt wird.

3. Verfahren nach Anspruch 1, dadurch gekennzeichnet, daß die Chlorwasserstoffsäure in das Reaktionsmedium bei einer Temperatur zwischen 50 und 180 °C eingeführt wird.

4. Verfahren nach einem der Ansprüche 1 bis 3, dadurch gekennzeichnet, daß das Molverhältnis der Chlorwasserstoffsäure zum Äthylenoxid zwischen dem stöchiometrischen und 40 liegt.

5. Verfahren nach Anspruch 4, dadurch gekennzeichnet, daß das Molverhältnis der Chlorwasserstoffsäure zum Äthylenoxid zwischen 2 und 20 liegt.

6. Verfahren nach einem der Ansprüche 1 bis 5, dadurch gekennzeichnet, daß das Reaktionsmedium auf einer Temperatur zwischen 130 und 300 °C gehalten wird.

7. Verfahren nach Anspruch 6, dadurch gekennzeichnet, daß das Reaktionsmedium auf einer Temperatur zwischen 130 und 250 °C gehalten wird.

8. Verfahren nach einem der Ansprüche 1 bis 7, dadurch gekennzeichnet, daß das Reaktionsmedium bei einem Druck unter 20 bar gehalten wird.